# EUROPÄISCHE PATENTSCHRIFT

(11) **EP 1 985 280 B1**
(45) Veröffentlichungstag und Bekanntmachung des Hinweises auf die Patenterteilung: **13.07.2016**
(21) Anmeldenummer: 08405099.6
(22) Anmeldetag: 07.04.2008
(51) Int. Cl.: A61Q 5/00, A61Q 7/00, A61Q 19/10, A61K 9/06, A61K 9/107, A61K 9/113, A61K 36/00, A61Q 19/00, A61Q 19/08, A61K 8/97, A61K 9/127, A61K 8/14

(54) **Kosmetisches Produkt zur topischen Anwendung für den Schutz und die Erneuerung von Hautstammzellen, welches sich von dedifferenzierten Pflanzenzellen ableitet**
Cosmetic product for topical use for protecting and renewing skin stem cells derived from dedifferentiated plant cells
Produit cosmétique destiné à l'application topique en vue de la protection et du renouvellement de cellules souches de la peau, qui résulte de cellules végétales dédifférenciées

(30) Priorität: 27.04.2007 CH 701072007
(43) Veröffentlichungstag der Anmeldung: 29.10.2008
(73) Patentinhaber: Mibelle AG, 5033 Buchs (CH)
(72) Erfinder: Blum, Peter, 8805 Richterswil (CH); Schürch, Cornelia, 5600 Lenzburg (CH); Schmid, Daniel, 5200 Brugg (CH); Zülli, Fred, Dr., 5024 Küttigen (CH)
(74) Vertreter: Rottmann, Maximilian

(56) Entgegenhaltungen:
- EP-A- 1 064 932
- EP-A- 1 174 120
- WO-A-01/47538
- WO-A-03/077881
- WO-A-2005/072697
- FR-A- 2 534 487
- US-A- 5 643 598
- US-B1- 6 555 118
- DATABASE EPODOC EUROPEAN PATENT OFFICE, THE HAGUE, NL; JP3277219 9. Dezember 1991 (1991-12-09), SUNTORY LTD: "Tissue Culture of Rose" XP002456156 & JP 03 277219 A (SUNTORY LTD) 9. Dezember 1991 (1991-12-09)

## Beschreibung

### Feld der Erfindung

Die vorliegende Erfindung befasst sich mit der Verwendung von dedifferenzierten Pflanzenzellen in kosmetischen Produkten zum Schutz von Stammzellen gegen intrinsische und extrinsische Stressfaktoren, insbesondere zur Förderung der Proliferation von Stammzellen und deren Schutz vor Apoptose (Zelltod). Die Erfindung bezieht sich im Besonderen auf die Verwendung von dedifferenzierten Pflanzenzellen aus den Früchten von *Malus domestica* (Apfel) der Sorte Uttwiler Spätlauber (*Malus domestica Cultivar Uttwiler Spätlauber).*

### Stand der Technik

Stammzellen (SZ) sind uniforme undifferenzierte Zellen mit den allgemeinen Eigenschaften sich konstant zu Erneuern und der einzigartigen Fähigkeit sich durch Teilung und Differenzierung in jeden anderen Zelltyp zu verwandeln. Durch dieses Potential sind SZ eine erneuerbare Quelle für menschliches Gewebe. SZ sind daher ein wichtiger Gegenstand der medizinischen Forschung geworden für verschiedene Anwendungen, wie Gentherapie, Organtransplantation, Diabetes, plastische Chirurgie.

SZ lassen sich in zwei Gruppen aufteilen: embryonische und adulte SZ. Embryonische Stammzellen (ES) spielen eine Schlüsselrolle in der ersten Entwicklungsphase eines Organismus. Sie sind zur unendlichen Teilungen befähigt und können jeden Gewebetyp ausbilden der benötigt wird. Sie sind deshalb in der Lage aus einer einzelnen Zelle einen ganzen Körper zu formen, sei es die jeweilige Pflanze oder das Tier von der sie herstammen. Durch diese Fähigkeit werden sie auch als pluripotente Zellen bezeichnet. Leider ist diese Fähigkeit beim Menschen auf das Embryo-Stadium beschränkt. In späteren Lebensphasen eines Subjekts sind ES nicht mehr länger vorhanden.

Der zweite Typ der Stammzellen sind adulte Stammzellen. Diese Zellen konnten bislang in vielen ausgewachsenen Geweben und Organen, wie dem Knochenmark, der Bauchspeicheldrüse, dem Rückgrat, dem Hirn, dem zentralen Nervensystem, dem peripheren Blut, dem Zahnmark, den Blutgefässen, den Skelettmuskeln, der Hornhaut, der Retina, der Leber, dem Nabelschnurblut, dem Herzen, dem Epithel des Verdauungstraktes, und der Haut identifiziert werden. Verglichen mit ES haben adulte SZ, die aus solchen Geweben stammen, nur eine beschränkte Auswahl in der Differenzierung. Einige können nur in einen Gewebetyp differenzieren, meistens den, welcher sie umgibt. Sie werden deshalb als unipotente SZ bezeichnet. Andere können in verschiedene Gewebetypen differenzieren und werden daher multipotent genannt.

Vielversprechend sind beide Typen der adulten SZ für medizinische Anwendungen, weil sie einfacher zugänglich und ethisch weniger problematisch in der Herstellung sind als ES. Einen Überblick über SZ und ihre Möglichkeiten kann in Lemoni et al., 2005, Stem Cell Plasticity: Time for a Reappraisal, Hematologica / The Hematology Journal, 90 (3), 360 bis 381, nachgelesen werden.

Die Haut von Säugern ist ein mehrschichtiges System, das sich ständig erneuert. Der Teil, welcher in direktem Kontakt zur Aussenwelt steht, nennt man Epidermis. Hauptaufgabe dieses spezialisierten Gewebes ist der Schutz des Körpers gegen Austrocknung, Verletzung und Infektionen. Es besteht aus vier verschiedenen Schichten, die alle von einem Zelltyp geformt werden, den so genannten Keratinozyten. Obschon dieser Zelltyp nicht sehr differenziert ist, hat er trotzdem seinen Ursprung in spezialisierten Hautstammzellen. Diese befinden sich in der untersten Schicht der Epidermis, der Basalschicht.

Mehrere Arbeiten haben bereits erfolgreich solche Hautstammzellen isoliert. Es konnte sogar gezeigt werden, dass Hautstammzellen in tieferen Geweben der Haut gefunden werden können und zwar in der so genannten Ausbuchtung des Haarfollikels. Diese SZ sind gegenüber SZ in der Basalschicht multipotent, d.h. befähigt sich in jeden Gewebetyp der Haut zu differenzieren. Ein Überblick kann nachgelesen werden bei: Roh et al., 2006, Cutaneous Stem Cells and Wound Healing, Pediatric Research, 59 (4), Pt 2, 100 bis 103R; Morasso et al., 2005, Epidermal Stem Cells; The Cradle of Epidermal Determination, Differentiation and Wound Healing, Biol. Cell., 97, 173 bis 183; sowie Alonso et al., 2003, Colloquium: Stem Cells of the Skin Epithelium, PNAS, 100, Suppl.1, 11830 bis 11835.

Hautstammzellen sind entscheidend in der Wundheilung, der Erneuerung von Haut und Haar. Die Kapazität dieser Fähigkeiten kann aber gestört werden durch genetische Probleme, Umwelteinflüsse oder durch den Alterungsprozess. Der Schutz dieser SZ ist deshalb äussert wichtig. Ziel der vorliegenden Erfindung war daher, wie weiter unten noch im Detail ausgeführt wird, einen pflanzlichen Extrakt zu entwickeln, der in kosmetischen Produkten diese SZ schützen und stimulieren kann.

Pflanzliche Extrakte und der Gebrauch von Pflanzenteilen wie Blätter, Früchte, Blumen, Stämme, Rinden, Blütenstände und Wurzeln für kosmetische und medizinische Anwendungen sind seit dem Altertum bekannt. Produkte davon können beispielsweise essentielle Öle, Fasern, Stärke, Aromen, Färbemittel, Antibiotika, Proteine, Phenole, Säuren oder Fette sein. Die Verwendung von Pflanzen oder pflanzlichen Extrakten in Kosmetika ist weit verbreitet. Es existiert eine grosse Anzahl von verschiedenen Verwendungen, wie beispielsweise Befeuchtung, Aufheller, Bräuner, Make-up, Sonnenfilter, Radikalfänger, Antioxidantien, Immunstimulierung, Detergenzien, Konservierungsmittel oder Verdickungsmittel. Beispiele von unlängst gefundenen Anwendungen sind beschrieben in: KR20040091178, KR20040059007, US20062400129, WO2006099930, WO2006086707, US2006153792, JP2006151934, WO2006068777, WO2006053761, WO2006008418, LV13345, UA73556, CN1679498 und vielen anderen

Das Spektrum von verschiedenen Pflanzen oder Pflanzenbestandteilen ist breit und beinhaltet beispielsweise Algen, Sukkulenten, Beeren, Fleischfressende Pflanzen, Kräuter, Getreide und Bäume. Übliche wohlbekannte Beispiele von verwendeten Pflanzen, die aber nicht nur auf diese beschränkt sind, sind Spirulina-Algen, Aloe Vera, Ringelblume, Ginkgo, Ginseng, Iris, Baldrian, Salbei, Lavendel, Thymian, Pfefferminze, Johanniskraut, Zitronen, Pfirsich, Guave, Avocado, Weizen und Hafer.

Es gibt jedoch Einschränkungen für die Verwendung von Pflanzen oder Pflanzenbestandteilen:
- Die Verfügbarkeit kann eingeschränkt sein beispielsweise durch Jahreszeiten, beschränkte Lagerbestände, Artenschutz, Probleme in der Kultivierung oder Missernten.
- Die Qualität ist nicht konstant, beispielsweise infolge jahreszeitlicher Veränderungen, verschiedener Kultivierungsmethoden, geographischer Unterschiede, unterschiedlicher Lieferanten, Klone, Umweltverschmutzung oder des physischen Status.

Diese Tatsachen verunmöglichen häufig den Einsatz von Pflanzen in kosmetischen Anwendungen.

Die Verwendung von Verfahren der Pflanzen-Zellkultur-Technik kann daher helfen solche Probleme zu lösen. Sie beinhaltet Techniken, die es ermöglichen unter Einhaltung gewisser bekannter Verfahrensschritte uniforme dedifferenzierte Zellen zu erhalten, welche folgende Vorteile gegenüber ganzen kultivierten Pflanzen aufweisen:
- Unabhängigkeit von Jahreszeiten;
- Kontinuierliche Produktion;
- Frei von Umweltverschmutzungen und anderen Unreinheiten;
- Kontrollierbare und reproduzierbare Produktion von Metaboliten im Bezug auf Quantität und Qualität;
- Schutz seltener oder beschränkter Pflanzenreserven;
- Keine Einschränkung der Marktverfügbarkeit.

Beispiele für die Verwendung von Pflanzen-Zellkulturen von verschiedenen Spezies, ihre Kultivierung und die Anwendung in kosmetischen Produkten sind beispielsweise zu finden in: EP1244464, FR2837385, US2006021084, WO2005108596, WO2005070066.

Die Grundlage der Kultivierung solcher dedifferenzierter Pflanzenzellen nützt den biologischen Umstand, dass jede Pflanzenzelle die Fähigkeit in sich trägt, die ganze Pflanze, von der sie herstammt zu bilden. Diese Fähigkeit nennt sich Totipotenz und ist vergleichbar mit der Pluripotenz von animalen ES. Deshalb ist anzunehmen, dass dedifferenzierte Pflanzenzellen einen positiven Einfluss haben auf den Schutz und die Aktivierung von Hautstammzellen.

Um diesen Effekt zu erreichen, können verschiedene dedifferenzierte Pflanzenzellen verwendet werden. Es lassen sich aber mit diesen Pflanzenextrakten noch weitere zusätzliche nützliche Effekte erzielen. Eine Nachforschung unter mehreren Pflanzengruppen zeigte, dass Äpfel oder Früchte, die der Unterfamilie *Maloideae* innerhalb der Rosaceae-Familie angehören, vielversprechend sind. Ein bekannter Vertreter dieser Familie ist der Kulturapfelbaum *(Malus domestica).* Äpfel haben eine lange Tradition in kosmetischen Anwendungen. Ursprünglich wurden sie als Masken aus gepresstem Fruchtfleisch oder Fruchtschalen angewendet, die für Feuchtigkeit und Straffheit der Haut sorgten. Eine andere Anwendung ist der Gebrauch von Aromen und Extrakten der Äpfel in allen Arten von kosmetischen Produkten, wie beispielsweise Shampoos, Lotionen, Seifen, Badezusätze oder Zahnpasten.

Die Hauptinhaltsstoffe von Äpfeln sind verschiedene Zucker, Vitamine, Säuren, Öle, Wachse und Polyphenole. Kürzlich erschienene Studien konnten beweisen, dass speziell die Gesamtpolyphenole in Extrakten von Äpfeln oder polyphenolreichen Apfelsäften helfen können in der Prävention und Behandlung von Dickdarmkrebs (Eberhart et al., 2000, Antioxidant Activity of Fresh Apples, Nature, 405, 903 bis 904; Liu et al., 2003, Antiproliferative Activity of Apples is not due to Phenolic-induced Hydrogen Peroxide Formation, J. Agric. Food Chem., 51, 1718 bis 1723; Kern et al., 2005, Inhibitors of the Epidermal Growth Factor Receptor in Apple Juice Extract, Mol. Nutr. Food Res., 49, 317 bis 328). Zu einem gewissen Anteil hatten dabei die polyphenol-reichen Säfte einen Einfluss auf den Wnt-Pathway. Dieser Pathway ist eine zellbiologische Signalkaskade in der das Hauptprotein β-Catenin ist. Unter normalen Umständen ist dieses Protein auf einem gleich bleibenden Niveau in der Zelle vorhanden. Ist dieses Niveau gestört, wie dies bei einer Krebszelle der Fall ist, steigt das Niveau von β-Catenin, und es wird in den Zellkern transportiert und initiiert dort die Transkription von Genen, welche zu unkontrollierter Teilung der Zelle führt. Kern et al. (2006, Modulation of Key Elements of the Wnt-Pathway by Apple Polyphenols, J. Agric. Food. Chem., 54, 7041 bis 7046) konnten zeigen, dass das Niveau von intrazellulärem β-Catenin in in-vitro-kultivierten Dickdarmkrebszellen durch die Verabreichung von Apfelsaft reduziert wurde.

Des Weiteren wurde gefunden, dass Äpfel eine grosse antioxidative Aktivität aufweisen und die antioxidative Kapazität im Blut erhöhen können (Rezk et al., 2002, The Antioxidant Activity of Phloretin: The Disclosure of a new Antioxidant Pharmacophore in Flavonoids, Biochem. Biophys. Res. Commun., 295, 9 bis 13; Lee et al., 2003, Major Phenolics in Apple and their Contribution to the Total Antioxidant Capacity, J. Agric. Food Chem., 51, 6516 bis 6520; Vrohovsek et al., 2004, Quantitation of Polyphenols in DifferentApple Varieties, J. Agric. Food Chem., 52, 6532 bis 6538; Lotito et al., 2004, Relevance of Apple Polyphenols as Antioxidants in Human Plasma: Contrasting in-vitro and in-vivo Effects, Free Rad. Biol. Med., 36, 201 bis 211; Bitsch et al., 2000, Bioavailability of Antioxidative Compounds from Brettacher Apple Juice in Humans, Food Sci. Emerg. Technol., 1, 245 bis 249). Aus diesem Grunde sind Äpfel äusserst interessant für die Gewinnung einer dedifferenzierten Zellkultur und deren anschliessenden Verwendung in kosmetischen Produkten.

Die dedifferenzierten Pflanzenzellen besitzen eine komplexe Matrix aus Bestandteilen von Salzen, Säuren, Polyphenolen, Zuckern, Fetten, Proteinen und anderen Stoffen. Neben den bekannten Stoffen ist auch eine unbekannte Fraktion von Bestandteilen vorhanden, die möglicherweise sehr wertvoll für kosmetische Anwendungen ist. Es ist bekannt, dass rohe Pflanzenextrakte häufig eine bessere Wirkung erzielen, als die identifizierten und isolierten Einzelkomponenten. Deshalb ist es sinnvoll, das ganze Zelllysat für eine Anwendung zu verwenden.

Um eine solche totale Fraktion aller Inhaltsstoffe zu erhalten, sind spezielle Techniken notwendig, da ein Teil davon wasserlöslich und der andere fettlöslich ist. Es wurde vorgeschlagen, Pflanzen-Zellkultur-Präparate mit der Hilfe von Gefriertrocknung zu verarbeiten (z.B. WO2005072697, US20050265953). Diese gefriergetrockneten Zellen wurden danach zermahlen und in topischen Produkten eingesetzt.

Da der Transport von Stoffen durch die Hautbarriere sehr limitiert ist, wurde die Technik zur Herstellung von Liposomen für zahlreiche kosmetische Anwendungen entwickelt (z.B. KR20050091162, KR920005639B, GB2415375, WO2004067012, EP1498420, US2002160064, AU2388099). Der Gebrauch dieser Technik ermöglicht eine bessere Penetration von Stoffen in tiefere Hautschichten. Ein weiterer Vorteil von Liposomen ist auch die Einkapselung von fettlöslichen Stoffen in die Membran und damit die Dispergierung in wässrigen Phasen. Die Techniken für die Herstellung von Liposomen sind verschieden. Hauptschritte der Produktion beinhalten das Lösen einer Phospholipid-Mischung in einem geeigneten Lösungsmittel (beispielsweise Glycerin oder Alkohol), Einmischen der gelösten Lipide in eine wässrige Phase, Einbringen von Energie (beispielsweise durch Rühren, Schütteln, Druck oder Hitze) zur Formung der Liposomen. Die verwendete Energieform kann, wie oben bereits erwähnt, Druck sein. Die Formung von Liposomen mittels Hochdruckhomogenisation ist eine bekannte Technik. Beispiele für pharmazeutische oder kosmetische Produkte sind beispielsweise in WO9949716, NZ502840, EP0782847 zu finden. Interessanterweise kann dieselbe Technik auch verwendet werden, um Zellen aufzuschliessen und deren Lysat zu gewinnen (z.B. DE19918619). Deshalb ist es möglich Pflanzenzellen aus Suspensionskulturen mittels Hochdruckhomogenisation aufzuschliessen und gleichzeitig die öl- und wasserlöslichen Wirkstoffe in leere Liposomen zu extrahieren. Damit kann die Stabilität der Wirkstoffe und deren Transport in die Haut verbessert werden.

Aus der oben erwähnten Veröffentlichung WO 2005/072697 A1 ist bereits die Verwendung von Lyophilisaten von dedifferenzierten Pflanzenzellen zur Depigmentierung der Haut bekannt. Sie setzt den Einsatz von Lyophilisaten von dedifferenzierten Pflanzenzellen, insbesondere Zellen von halophilen Pflanzen, voraus. Ein Einsatz zur Stimulierung und zum Schutz von Hautstammzellen ist nicht vorgesehen.

Aus der Veröffentlichung EP 1 174 120 A1 ist sodann die Verwendung von Extrakten, insbesondere Lyophilisaten, von dedifferenzierten Zellen von Pflanzen der Familie *Iridaceae* (Irisgewächsen) zur Immunstimulanz bekannt. Andere Pflanzen oder Verwendungen werden nicht vorgeschlagen.

In der Veröffentlichung EP 1 064 932 A1 wird die Verwendung von Extrakten von dedifferenzierten Pflanzenzellen in Deodorantien vorgeschlagen. Andere Verwendungen werden nicht genannt.

Aus der Veröffentlichung WO 03/077881 A2 ist die Verwendung von auf komplizierte Weise erhaltenen Lyophilisaten von Metaboliten von dediffernzierten Zellen der Rebe für die Herstellung von Kosmetika bekannt. Sie setzt den Einsatz von Lyophilisaten voraus, und ein Einsatz zur Stimulierung und zum Schutz von Hautstammzellen ist nicht vorgesehen. Auch werden andere Pflanzenarten nicht erwähnt.

Weiter sieht die Veröffentlichung WO 01/47538 A1 den Einsatz von Extrakten von dedifferenzierten Zellen von Pflanzen der Gattung *Leontopodium* (Edelweiss) als UV-Filter vor. Andere Pflanzen oder Verwendungen werden nicht vorgeschlagen.

### Aufgabe der Erfindung

Aufgabe der Erfindung war nun die Schaffung eines kosmetischen Produktes, welches Stammzellen gegen intrinsische und extrinsische Stressfaktoren schützt, insbesondere deren Proliferation fördert und sie vor Apoptose (Zelltod) schützt.

Diese Aufgabe wird gelöst durch die Verwendung eines Extraktes aus einer Suspension von dedifferenzierten Pflanzenzellen gemäß der Anspruch 1, aus Pflanzen der Unterfamilie *Maloidae* (Kernobstgewächse), und vorzugsweise von Pflanzen von *Malus domestica Cultivar Uttwiler Spätlauber* (Apfel Sorte Uttwiler Spätlauber), einer alten und seltenen Apfelsorte.

### Ausführung der Erfindung

Die Herstellung geeigneter Extrakte umfasst die folgenden Hauptschritte:
a) Erstellung einer stabilen dedifferenzierten Zelllinie im Labormassstab;
b) Massenkultivierung der Zellen in einem Einwegbeutel-Reaktorsystem (sog. Wave-Reaktor); und
c) Herstellung eines Total-Extraktes mittels Hochdruckhomogenisation unter Verwendung von leeren Liposomen.

Zweckmässigerweise folgt man dabei beim Schritt c) dem folgenden Verfahrensablauf:
- Aufschluss der Pflanzenzellen durch Hochdruckhomogenisation;
- Extraktion und Stabilisierung von Inhaltsstoffen mit Liposomen;
wobei beide Teile des Verfahrensablaufs in einem einzigen Verfahrensschritt gleichzeitig durchgeführt werden.

### Detaillierte Beschreibung der Erfindung

### Induktion und Stabilisierung der Zelllinie (a)

Folgende Schritte führen zu einer dedifferenzierten Zellline aus pflanzlichem Gewebe:
(a1) Auswahl eines geeigneten Gewebes für die Induktion;
(a2) Oberflächensterilisation;
(a3) Plattieren der Explantate auf einem geeigneten Festmedium zur Kallusinduktion;
(a4) Ernte des Kallus, der auf der verletzten Oberfläche der Explantate gewachsen ist;
(a5) Subkultivierung des so gewonnenen Kallus auf demselben Medium bis die Zellen voll dedifferenziert sind;
(a6) Einbringen der dedifferenzierten Zellen in ein geeignetes Flüssigmedium;
(a7) Homogenisation der Zellen in Suspension, bis keine grossen Zellklumpen mehr vorhanden sind; und
(a8) Subkultivierung und kontinuierliche Charakterisierung der Zellsuspension.

Grundlegende Arbeitsprotokolle für Pflanzenzellkulturen können der Standard-Literatur entnommen werden (z.B. Plant Cell Culture: A Practical Approach, Editor P.A. Dixon, 1994, Oxford University Press). Protokolle für die Arbeit und geeignete Medien für die Initiierung von Pflanzenzellkulturen aus Äpfeln sind beschrieben von Nitsch et al., 1970, Bases physiologiques de la production de chair de pomme et de poire in vitro, Bull. Soc. Bot. Fr., 117, 479 bis 492; und Pech et al., 1975, Croissance in vitro de tissues et de spensions cellulaires de pomme, Bull. Soc. Bot. Fr., 122, 183 bis 194. Gemäss diesen Protokollen sollte die Initiierung, die Selektion und die Erhaltung von solchen Kulturen für den Fachmann kein Problem darstellen.

### Biomasse-Produktion (b)

In einem weiteren Verfahren wird die so gewonnene Suspensionskultur über mehrere kontinuierliche Schritte von kleinen Laborflaschen (Erlenmayerflaschen mit üblicherweise 200 ml Inhalt) zum Produktionsmassstab von 50 bis 100 Liter weitergezüchtet. In diesem Prozess werden 5 bis 20 % (bevorzugt 10 %) des nächstgrösseren Kultivierungsvolumens von einer voll durchgewachsenen Zellsuspension als Einsaat (Inokulum) verwendet. Die Massstabsvergrösserung kann z.B. in Schritten von 0,1 / 1 / 10 / 100 Liter erfolgen.

Bei Kultivationsvolumen über 1 Liter wird der Einsatz von speziellen Bioreaktoren anstatt der zuvor verwendeten Kulturflaschen notwendig. Es sind viele verschiedene Systeme auf dem Markt erhältlich. Die Kultivierung kann, ist aber nicht limitiert auf diese, in Rührreaktoren, Blasensäulen, Schlaufenreaktoren oder neu entwickelten Einwegsystemen erfolgen, welche für die Kultivierung von Pflanzenzellkulturen geeignet sind. Kritisch für alle diese Kulturen ist der Einfluss von Scherstress, welcher die Kulturen schädigen kann. Darum ist der wichtigste Parameter für die Selektion eines geeigneten Reaktorsystems üblicherweise die Art und Weise der Homogenisation der Kultur.

Weiterhin ist die Überwachung der Kultur sehr wichtig. Im Vergleich zu Hefe- oder Bakterien-Kulturen ist die Messung der Biomasse schwierig und der Zuwachs an Biomasse muss durch indirekte Parameter, wie beispielsweise dem Verbrauch an Kohlenstoff, dem Absinken der Leitfähigkeit, dem pH-Wert oder dem Zuwachs der optischen Dichte erfasst werden. Ist einmal eine solche Kontrolle etabliert, kann der Endpunkt resp. der Erntezeitpunkt der Kultivierung festgelegt werden.

Wichtig ist ebenso die Überprüfung und Analyse von Sekundärmetaboliten, welche charakteristisch für die Zellkultur sind. Die Messung solcher Stoffe kann mittels HPLC-VIS/UV/MC, LC, GC-MS, beispielsweise enzymatisch oder optisch erfolgen. Entscheidend ist die stabile und kontinuierliche Expression von solchen Metaboliten während des ganzen Prozesses.

### Aufarbeitung der Biomasse (c)

Um einen Extrakt zu erhalten, der die ganze Essenz der kultivierten Zellen enthält werden die Zellen mit Liposomen aufgeschlossen. Hauptbestandteil dieses Verfahrens ist die Anwendung der Hochdruckhomogenisation auf die ganze Zellbrühe zusammen mit einem Liposomen-Präparat. Der grosse Vorteil dieses Verfahrens ist die einfache und kostengünstige Handhabung.

Das Verfahren umfasst im Einzelnen die folgenden Schritte:
(c1) Zugabe eines geeigneten Liposomen Präparates zur Zellbrühe;
(c2) Zugabe eines geeigneten Konservierungsmittels;
(c3) Zugabe geeigneter Antioxidantien;
(c4) Mischen der Substanzen; und
(c5) Hochdruckhomogenisation.

Als Konservierungsmittel können alle für Kosmetika zugelassenes Konservierungsmittel natürlichen oder synthetischen Ursprungs, wie beispielsweise Phenoxyethanol, Benzoesäure, Propionsäure, Alkohol oder Silberchlorid, verwendet werden.

Um den Extrakt zusätzlich vor Oxidation zu schützen, können Antioxidantien, wie beispielsweise Ascorbinsäure oder Tocopherol, beigemengt werden.

Bei dem beschriebenen Verfahren ist es auch möglich, noch weitere Substanzen, die im Präparat oder im kosmetischen Produkt von Nutzen sind, beizumengen. Nachdem alle Komponenten zusammengefügt wurden, muss die Mischung verrührt werden, um das Konservierungsmittel, sowie andere Zutaten zu lösen. Das kann beispielsweise mit einem Blattrührer, einem Homogenisierungsstab oder durch Pumpen durch statische Mischelemente geschehen.

Die nachfolgende Hochdruckhomogenisation verfolgt zwei Ziele:
- Zerstörung der Zellmembranen, damit die extrahierbaren Stoffe freigesetzt werden, und
- Generierung von fein dispergierten Liposomen, welche die fettlösliche und wasserlöslichen Fraktion der Zellen enthalten.

Geeignete Hochdruckhomogenisatoren sind kommerziell auf dem Markt erhältlich. Das Prinzip der Reaktionskammer muss aus verschiedenen Möglichkeiten ausgewählt und vorgängig getestet werden. Ebenso muss die Anzahl der Durchläufe durch die Reaktionskammer bis alle Zellwände aufgeschlossen oder eine gewünschte Homogenität des Extraktes erreicht ist, getestet werden.

Der so erhaltene Extrakt kann danach direkt in ein kosmetisches Produkt, wie beispielsweise Cremen, Seifen, Lotionen, Gele oder Haarseren, eingearbeitet werden. Falls der Extrakt als Halbfabrikat dienen soll, ist eine zusätzliche Verdickung denkbar. Als Verdickungsmittel können dabei alle für Kosmetika erlaubten natürlichen und synthetischen Verdicker, wie beispielsweise Xanthangummi, Hyaluronsäure, Carrageen, Dextrin, modifizierte Stärke oder Agar, eingesetzt werden.

### Abbildungen

In den beigefügten Abbildungen zeigen:

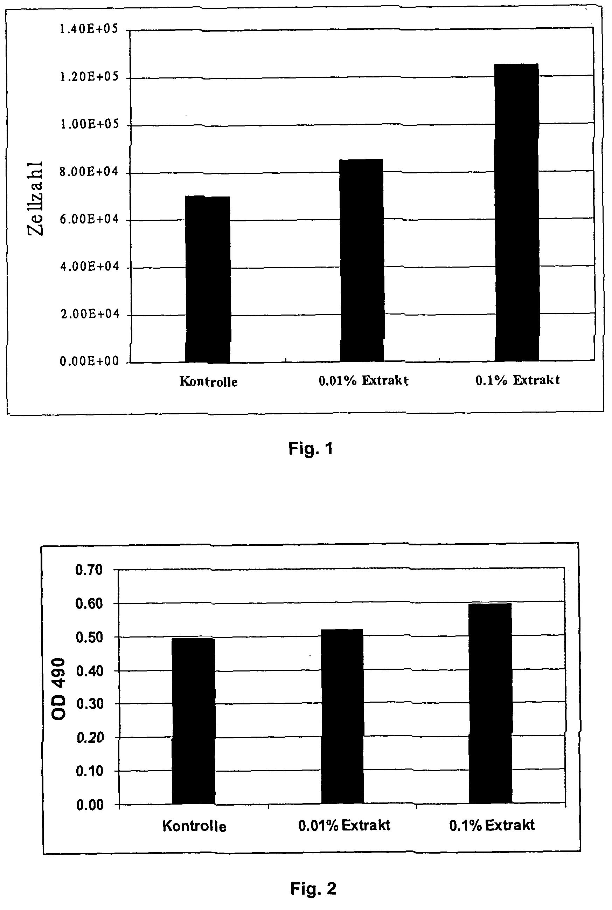

Fig. 1 zeigt den Zuwachs in der Zellzahl von Nabelschnurstammzellen in Abhängigkeit von unterschiedlichen Konzentrationen eines liposomalen Extraktes der aus dedifferenzierten Zellen von Äpfeln der Sorte Uttwiler Spätlauber stammte. Für die Studie wurde der Extrakt zentrifugiert und filtersterilisiert.
Fig. 2 zeigt den Zuwachs in der Proliferationsfähigkeit in einem MTS-Assay von Nabelschnurstammzellen in Abhängigkeit von unterschiedlichen Konzentrationen eines liposomalen Extraktes der aus dedifferenzierten Zellen von Äpfeln der Sorte Uttwiler Spätlauber stammte. Für die Studie wurde der Extrakt zentrifugiert und filtersterilisiert.

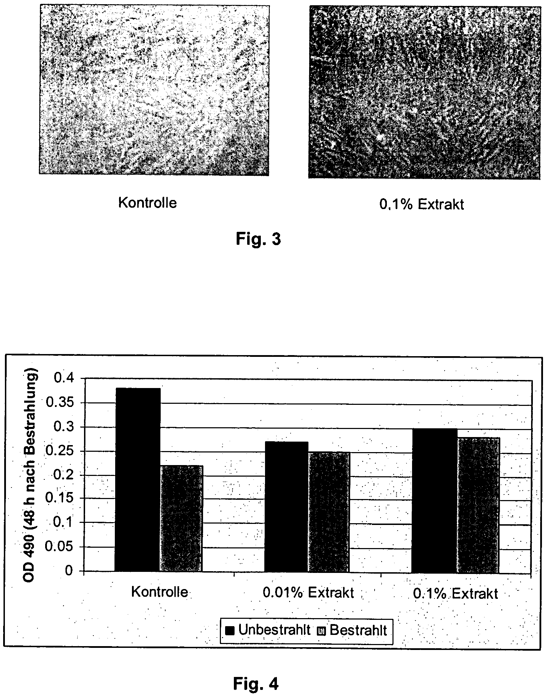

Fig. 3 zeigt mikroskopische Aufnahmen von Nabelschnurstammzellen. Die linke Fotografie zeigt dabei Zellen, welche in Medium ohne Extrakt gewachsen sind, die rechte Fotografie solche, die zusammen mit 0,1 % eines liposomalen Extraktes der aus dedifferenzierten Zellen von Äpfeln der Sorte Uttwiler Spätlauber stammte, kultiviert wurden. Für die Studie wurde der Extrakt zentrifugiert und filtersterilisiert.
Fig. 4 zeigt die Proliferationsfähigkeit von Nabelschnurstammzellen einer Kontrolle, sowie eines liposomalen Extraktes unterschiedlicher Konzentration, der von dedifferenzierten Zellen von Äpfeln der Sorte Uttwiler Spätlauber stammten, 48 h nach einer Bestrahlung mit UV. Für die Studie wurde der Extrakt zentrifugiert und filtersterilisiert.

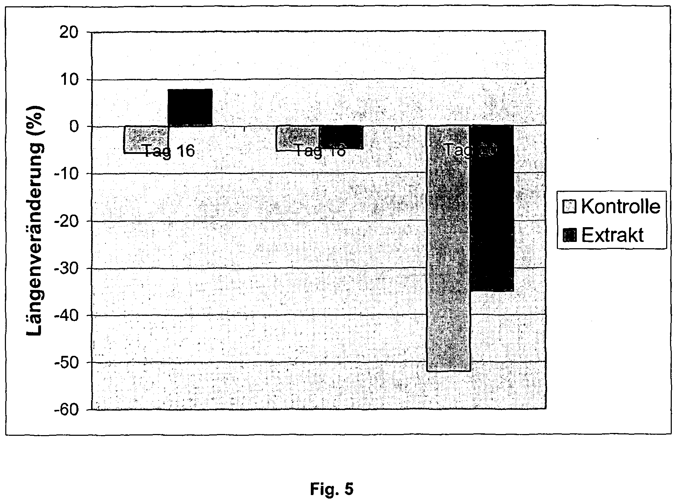

Fig. 5 zeigt den zeitlichen Einfluss eines liposomen Extraktes gemäss Beispiel 10 auf die Länge von Haarfolikeln.

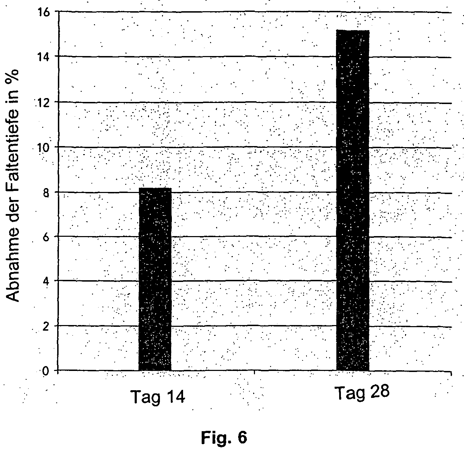

Fig. 6 zeigt die Wirkung einer erfindungsgemässenen Zubereitung als Antifaltencreme im nachstehend beschriebenen Test 1.

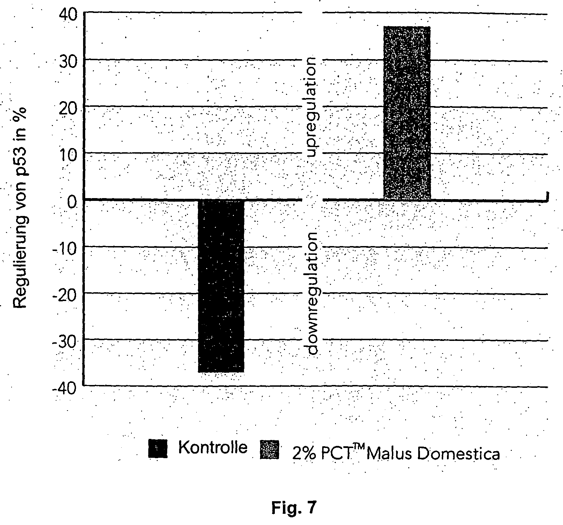

Fig. 7 zeigt die Wirkung einer erfindungsgemässen Zubereitung auf eine gestresste Haut im nachstehend beschriebenen Test 2.

In den Fig. 2 und 4 steht die Abkürzung "OD" für "Optische Dichte".

### Beispiele

### Beispiel 1

### Gewinnung einer dedifferenzierten pflanzlichen Zellkultur

Ausgereifte Äpfel der Sorte Uttwiler Spätlauber wurden mit Leitungswasser gespült. Zylindrische Stücke von etwa einem Zentimeter Durchmesser wurden mit einem Korkbohrer entlang der Achse des Kerngehäuses ausgestochen. Die Zylinder wurden für die Oberflächensterilisation für 30 Sekunden in 70 % Ethanol und danach für 10 Minuten in 2,5 % Natriumhypochlorid mit 0,1 % des Netzmittels Tween 40 getaucht. Die sterilisierten Zylinder wurden danach dreimal mit destilliertem Wasser gewaschen, in Scheiben von etwa drei Millimeter Dicke geschnitten und auf Festmedium mit folgender Zusammensetzung pro Liter platziert:

| | | |
|---|---|---|
| Calciumchlorid | 332 | mg |
| Kaliumdihydrogenphosphat | 170 | mg |
| Kaliumnitrat | 1900 | mg |
| Magnesiumsulfat | 180,54 | mg |
| Ammoniumnitrat | 1650 | mg |
| Kobaltchloridhexahydrat | 0,025 | mg |
| Kupfersulfatpentahydrat | 0,025 | mg |
| Eisen-Natrium-EDTA | 36,7 | mg |
| Borsäure | 6,2 | mg |
| Kaliumiodid | 83 | mg |
| Mangansulfathydrat | 16,9 | mg |
| Dinatriummolybdatdihydrat | 0,25 | mg |
| Zinksulfatheptahydrat | 8,6 | mg |
| myo-Inositol | 100 | mg |
| Nikotinsäure | 5 | mg |
| Glycin | 2 | mg |
| Pyridoxinhydrochlorid | 0,5 | mg |
| Thiamidinhydrochlorid | 0,5 | mg |
| Folsäure | 0,5 | mg |
| Biotin | 0,05 | mg |
| Ascorbinsäure | 50 | mg |
| Thioharnstoff | 25 | mg |
| L-Asparagin | 180 | mg |
| Saccharose | 30000 | mg |

Der pH-Wert wurde mit Natronlauge auf 5,6 eingestellt. Agar wurde als Geliermittel in einer Konzentration von 0.8 % zugesetzt. Alle Zutaten wurden zusammengemischt und bei 121°C während 15 Minuten sterilisiert.

Die Induktion des Primärkallus erfolgte im Dunkeln bei 25°C. Die gebildeten Kalli wurden alle zwei bis drei Wochen geerntet und auf demselben Medium weiter inkubiert. Mehre Subkultivierungen folgten, bis der Kallus voll dedifferenziert war.

### Beispiel 2

### Gewinnung einer Suspensionskultur

Dedifferenzierte weiche Zellklumpen, welche auf dem Festmedium wuchsen, wurden genommen, homogenisiert und in dasselbe Medium ohne Geliermittel eingebracht. Dies ergab dann eine fein dispergierte Suspension, welche weiter für die Inokulation von grösseren Kultivierungssystemen verwendet werden konnte. Die Suspensionen wurden im Dunkeln bei 25 °C und einer Schüttelgeschwindigkeit von rund 100 rpm gezogen.

### Beispiel 3

### Massenvermehrung

Ein Zehntel einer voll durchgewachsenen Kultur (Zellanteil entspricht etwa 50 % des Totalgewichtes der Kultur) wurde für die Aussaat zum nächsten Volumenschritt verwendet. Die Massstabsvergrösserung erfolgte in einem Einwegbeutel-Reaktorsystem, das von Wave Biotech AG, Tagelswangen, Schweiz, stammte (sog. Wave-Reaktor). Die Vergrösserung erfolgte in den Schritten 1 / 10 / Litern. Die Temperatur wurde auf 25 °C eingestellt, die Belüftung auf etwa 0,1 vvm und verschiedenen Mischgeschwindigkeiten wurden verwendet je nach Beutel. Die Kultivierung erfolgte im Dunkeln und nahm etwa 20 Tage in Anspruch, bis ein Beutel komplett durchwachsen war.

### Beispiel 4

### Herstellung eines liposomalen Extraktes

Die ganze Zellbrühe wurde nach der Kultivierung mit einer Dispersion, welche leere Liposomen einer Grösse von rund 50 nm enthielt, gemischt. Die Mischung wurde danach viermal hochdruckhomogenisiert bei einem Druck von 1200 bar (1,2·10⁸ N m⁻²), was einen fein dispergierten Extrakt ergab.

### Beispiel 5

### Tagescreme

Die Prozentangaben beziehen sich auf die Gesamtmenge (w/w).

| | | |
|---|---|---|
| Ölphase 1: | Alkylbenzoate | 10 % |
| | Dimeticone | 3 % |
| | Archidylglycoside | 3 % |
| | Myristylglycoside | 2 % |
| Ölphase 2: | Polyacrylamide | 1 % |
| Wasserphase: | Entmineralisiertes Wasser | 71 % |
| | Glycerin | 5 % |
| | Phenoxyethanol | 1 % |

Die Ölphase 1 und die Wasserphase wurden auf 80 °C erhitzt und vermengt. Das Gemisch wurde auf 60 °C abgekühlt, die Ölphase 2 wurde dazugeben und es wurde gemischt. Die Mischung wurde auf 30 °C abgekühlt, es wurden 4 % des in Beispiel 4 beschriebenen Extraktes beigeben und es wurde erneut gemischt.

### Beispiel 6

### Flüssigbalsam für Kopfhaut

Die Prozentangaben beziehen sich auf die Gesamtmenge (w/w).

| | |
|---|---|
| Ethanol | 0,5 % |
| Urea | 5 % |
| Propylenglykol | 0,5 % |
| Carbomer | 0,4 % |
| Bisabolol | 0,1 % |
| PEG-60 | 0,6 % |
| D-Panthenol 75 % | 0,5 % |
| Natriumhydroxid 30 % | 0,4 % |
| Pflanzenzellextrakt aus Beispiel 4 | 1 % |
| Wasser | auf 100 % auffüllen |

### Beispiel 7

Die Prozentangaben beziehen sich auf die Gesamtmenge (w/w).

| *Phase* | *Stoff* | *Menge* |
|---|---|---|
| Wasserphase 1 (W1) | Wasser | auf100 % auffüllen |
| | Zitronensäure | 0,6 % |
| | Natriumbenzoat | 0,5 % |
| Wasserphase 2 (W2) | D-Panthenol 75 % | 0,7 % |
| Ölphase 1 (O1) | Cetearylalkohol | 4,5 % |
| | Dicocoylethyl-hydroxyethlmonium-methosulfat | 3 % |
| | Distearoylethyl-hydroxetylmonium-methosulfat | 1,5 % |
| | Dicapryrylether | 1 % |
| | Glycerinstearat | 1 % |
| Ölphase 2 (02) | Aminodimethicon | 0,3 % |
| Pflanzenextrakt (A) | Pflanzenzellextrakt aus Beispiel 4 | 2 % |

### Ausführung:

Wasserphase 1 mischen, auf 75 °C erwärmen und kurz vor dem Mischen mit der Ölphase 1 Wasserphase 2 (Panthenol) zugeben. Ölphase 1 auf 75 °C erwärmen, kurz vor dem Mischen mit den vereinigten Wasserphasen Ölphase 2 (Aminodimethicon) zugeben. Vereinigte Wasser- und Ölphasen mischen und homogenisieren. Auf 30 °C abkühlen und Phase A (Pflanzenextrakt) zugeben.

### Beispiel 8

### Augencreme

Prozentangabe bezieht sich auf die Gesamtmenge (w/w).

| *Phase* | *Stoff* | *Menge* |
|---|---|---|
| Wasserphase (W) | Wasser | auf 100 % auffüllen |
| | Zitronensäure | 0,6 % |
| | Glycerin | 5 % |
| | Butylenglycol | 5 % |
| | Galactoarabinan | 0,3 % |
| | Parabens in Phenoxyethanol | 0,8 % |
| Ölphase 1 (O1) | Polyglyceryl-3-methylglucosedistearat | 2,5 % |
| | Hydrogeniertes Polyisobuten | 3 % |
| | Pflanzliches Öl | 4 % |
| | Dicapryrylether | 3 % |
| | Behenylalkohol | 2 % |
| | Dimethicon | 0,5 % |
| Ölphase 2 (02) | Maisphosphate | 1 % |
| | Dimethicon | 0,5 % |
| Pflanzenextrakt (A) | Pflanzenzellextrakt aus Beispiel 4 | 2 % |

### Ausführung:

Wasserphase mischen und auf auf 80 °C erhitzen. Ölphase 1 auf 80 °C erhitzen, dann Ölphase 2 zugeben. Anschliessend vereinigte Ölphasen und Wasserphase vermischen und homogenisieren. Auf 30 °C abkühlen und Phase A (Pflanzenextrakt) zugeben und erneut mischen.

### Beispiel 9

### In-vitro-Test an Stammzellen

Der Test wurde an Stammzellen durchgeführt, welche aus der Nabelschnur stammten. Die Zellen wurden in einem Komplexmedium mit 10 % fötalem Kälberserum gezogen. Für den Test wurde nur der Überstand des Extraktes ohne die Zelltrümmer verwendet. Vor den Tests, wurde der Extrakt filtersterilisiert

Der Zusatz von 0.1 % des Extraktes resultierte in einem Zuwachs von rund 44 % in der Zellzahl (Fig. 1 und 3).

Ebenso konnte in einem nachfolgenden MTS-Assay eine Steigerung der Proliferationsfähigkeit der Zellen um 20 % bei 0,1 % Zusatz des Extraktes nachgewiesen werden (Fig. 2).

Neben den Wachstums- und Proliferationsstudien, wurden Nabelschnurzellen zusammen mit einem liposomalen Extrakt von dedifferenzierten Zellen von Äpfeln der Sorte Uttwiler Spätlauber auf die Folgen einer UV-Bestrahlung hin getestet. Der Einsatz von 0,1 % des Extraktes führte bei der Proliferationsfähigkeit lediglich zu einer Reduktion von rund 7 %, wohingegen die Kontrolle einen Proliferationsverlust von 42 % aufwies (Fig. 4).

### Beispiel 10

### Ex-vivo-Test an isolierten Haarfollikeln

Das Epithel der Haarwurzel beult zu einem suprabasalen Wulst aus, der die Nische der Keratinozyten-Stammzellen darstellt. Diese bestehen aus klonalen Subpopulationen, die Haut und Haarfollikel regenerieren. Isolierte Haarfollikel sind demnach ein geeignetes Modell, zur Untersuchung der Lebenserwartung von Stammzellen.

Von Hautmaterial, das von einer Schönheitsoperation stammt, wurden Haarfollikel isoliert. Die Follikel wurden anschliessend in eine Nährlösung gebracht, wo sie weiterlebten und anfingen zu wachsen. Haarfollikel können so normalerweise ungefähr 14 Tage am Leben erhalten werden. Danach beginnen die Zellen abzusterben und das neu gebildete Haar beginnt zu schrumpfen. Jeweils 12 Follikel wurden als Kontrollansatz nur in Nährlösung inkubiert und eine zweite Serie von 12 Follikeln wurde in Nährlösung mit 0,2 % liposomalem Extrakt von dedifferenzierten Zellen von Äpfeln der Sorte Uttwiler Spätlauber inkubiert. An den Tagen 16, 18 und 20 wurden die Längen der Haarfollikel gemessen.

Der ex-vivo-Test zeigte, dass die Follikel des Kontrollansatzes wie erwartet schon am Tag 16 ungefähr 6 % an Länge eingebüsst haben. Ein ähnliches Schrumpfen war am Tag 18 festzustellen. Am Tag 20 war dann ein deutliches Absterben von 52 % messbar. Die Follikel, die mit dem Extrakt behandelt wurden, blieben länger in der Wachstumsphase. Am Tag 16 konnte noch eine Längenzunahme von 8 % gemessen werden. Erst am Tag 18 zeigte sich ein leichtes Schrumpfen. Das Absterben am Tag 20 war deutlich geringer wie im Kontrollansatz.

Im Einzelnen ergaben sich folgende Werte der Längenveränderung, welche in Fig. 5 graphisch dargestellt sind:

| | Kontrolle | Extrakt |
|---|---|---|
| Tag 16 | -5,7 % | 7,8 % |
| Tag 18 | -5,3 % | -4,8 % |
| Tag 20 | -52 % | -35 % |

Das Beispiel 10 zeigt somit, dass ein liposomaler Extrakt von dedifferenzierten Zellen von Äpfeln der Sorte Uttwiler Spätlauber die Lebenserwartung von Keratinozyten-Stammzellen verlängern kann.

### Dermatologische Tests

### TEST 1

### Antifalten-Effekt von PhytoCellTec™ Malus Domestica

Der folgende dermatologische Test wurde ausgeführt von Dr. H. P . Nissen, Derma Consult GmbH, D-53347 Alfter, Deutschland. *PhytoCellTec™ Malus Domestica* ist der Markenname der Anmelderin für Produkte gemäss der vorliegenden Erfindung.

### Testprodukt

• Creme enthaltend 2.0 % *PhytoCellTec™ Malus Domestica*

### Testgebiet

• Augenfältchen (sog. Krähenfüsse)

### Probanden

- Anzahl Personen: 20
- Alter: 37 bis 64 Jahre
- Geschlecht: weiblich

### Anwendung

- Dauer: 28 Tage
- Häufigkeit: zweimal täglich

### Test-Parameter

• *Faltentiefe* gemessen mittels eines Apparates PRIMOS^{®} 5.5 der GFMesstechnik GmbH, D-14513 Teltow, Deutschland

### Durchführung der Untersuchung

### • Tag 0

Bestimmung des Testparameters im Testgebiet. Erste Anwendung des Testproduktes

### • Tag 1 bis 13

Anwendung des Testproduktes zweimal täglich

### • Tag 14

Bestimmung des Testparameters 8 bis 12 Stunden nach der letzten täglichen Anwendung des Testproduktes

### • Tag 15 bis 27:

Anwendung des Testproduktes zweimal täglich

### • Tag 28:

Bestimmung des Testparameters 8 bis 12 Stunden nach der letzten täglichen Anwendung des Testproduktes

### Resultate

Die zweimal tägliche Anwendung der Testcreme, welche 2 % *PhytoCellTec™ Malus Domestica* enthielt, während 28 Tagen führte zu einer signifikanten Abnahme der Faltentiefe bei allen getesteten Probanden. Diese Resultate sind in Fig. 6 dargestellt.

### TEST 2

### Wirkung von PhytoCellTec™ Malus Domestica auf gestresste Haut

Der folgende dermatologische Test wurde ausgeführt von F. Juchaux, of BlOalternatives, F-86160, Frankreich. *PhytoCellTec™ Malus Domestica* ist der Markenname der Anmelderin für Produkte gemäss der vorliegenden Erfindung.

### Einleitung

In normaler Haut wird das Tumorsuppressions-Gen p53 durch verschiedene Stressfaktoren upreguliert, z.B. infolge DNA-Schädigung (induziert durch UV- oder IR-Strahlung oder chemische Wirkstoffe, wie Wasserstoffperoxid), oxidativen Stress, oder osmotischen Schock. Das Protein p53 spielt im Zellzyklus eine wichtige Rolle als Transkriptionsregulator. In alter Haut wird dieses Gen durch Stress nicht mehr upreguliert sondern downreguliert.

### Testprodukt

### 2,0 % PhytoCellTec™ Malus Domestica

### Zellen

• Normal menschliche Hautfibroblasten (NHDF), welche nach der 10. Passage eingesetzt wurden

### Durchführung der Untersuchung

Die Fibroblasten wurden während 2 Stunden mit einem Kulturmedium, welches 600 µmol H₂O₂ enthielt, gestresst. Danach wurden die Zellen während 72 Stunden mit einem Medium, welches 2 % *PhytoCellTec™ Malus Domestica* enthielt, oder nur mit reinem Medium (Kontrolle), inkubiert. Nach der Inkubationszeit wurde die mRNA extrahiert und über eine Reverse-Transkription in cDNA transkribiert. Diese markierte cDNA wurden mit einem für spezifischen Minichip hybridisiert und die Menge an ³³P-markierten Genen gemessen. Diesr Minichip enthielt zirka 150 spezifische Gene, die für die Hautalterung verantwortlich sind.

### Resultate

In H₂O₂-gestressten Fibroblasten wurde das Tumorsuppressions-Gen p53 downreguliert. Mit 2 % *PhyfoCellTec™ Malus Domestica* behandelte H₂O₂-gestresste Zellen zeigten dagegen eine Upregulierung von p53. Diese Resultate sind in Fig. 7 dargestellt.

## Patentansprüche

1. Kosmetisches Produkt, enthaltend einen Extrakt aus einer Suspension aus dedifferenzierten Pflanzenzellen aus Pflanzen der Unterfamilie *Maloidae* (Kernobstgewächse), vorzugsweise von *Malus domestica Cultivar Uttwiler Spätlauber* (Apfel der Sorte Uttwiler Spätlauber) zur Behandlung von Stammzellen zum Schutz vor Apoptose und zur Förderung der Proliferation, wobei (1) der Extrakt durch Hochdruckhomogenisation eines Total-Extraktes gewonnen wird und (2) die Inhaltsstoffe mit Liposomen extrahiert und stabilisiert werden, wobei beide Teile des Verfahrensablaufs in einem einzigen Verfahrensschritt gleichzeitig durchgeführt werden.

2. Kosmetisches Produkt nach Anspruch 1, des weiteren enthaltend Konservierungsmittel und Antioxidantien.

3. Kosmetisches Produkt nach Anspruch 2, wobei das Konservierungsmittel ausgewählt wird aus Phenoxyethanol, Benzoesäure, Propionsäure, Alkohol, Silberchlorid.

4. Kosmetisches Produkt nach Anspruch 2, wobei das Antioxidant ausgewählt wird aus Ascorbinsäure, Tocopherol.

5. Kosmetisches Produkt nach einem der Ansprüche 1 bis 2, wobei das kosmetische Produkt einen Gehalt an Pflanzenzellsuspension von 0,01 bis 100 Gew.-% aufweist.

6. Kosmetisches Produkt nach Anspruch 5, wobei das kosmetische Produkt einen Gehalt an Pflanzenzellsuspension von 0,1 bis 10 Gew.-% aufweist.

7. Kosmetisches Produkt nach einem der vorherigen Ansprüche, in Form von Cremes, Seifen, Lotionen, Gelen oder Haarseren.

8. Kosmetisches Produkt nach einem der vorherigen Ansprüche, wobei das kosmetische Produkt einen Extrakt enthält, der ein Halbfabrikat ist.

## Claims

1. Cosmetic product containing an extract of a suspension of dedifferentiated plant cells of plants of the sub-family of *Maloidae* (Pome fruit), preferably from plants of *Malus domestica Cultivar Uttwiler Spaetlauber* (apples of the Cultivar Uttwiler Spaetlauber) for the treatment of stem cells for the protection against apoptosis and for stimulation of the proliferation, whereby (1) the extract is obtained from high pressure homogenisation of a total-extract and (2) the ingredients are extracted and stabilized by means of liposomes, whereby both steps of the process are simultaneously carried out in one single step.

2. The cosmetic product as set forth in claim 1, further containing preservative agents and antioxidants.

3. The cosmetic product as set forth in claim 2, whereby the preservative agent is selected from the group consisting of phenoxy ethanol, benzoic acid, propionic acid, alcohol, silver chloride.

4. The cosmetic product as set forth in claim 2, whereby the antioxidant is selected from ascorbic acid, tocopherol.

5. The cosmetic product as set forth in one of the claims 1 to 2, whereby the cosmetic product comprises 0.01 to 100 % by weight of the plant cell suspension.

6. The cosmetic product as set forth in claim 5, whereby the cosmetic product comprises 0.1 to 10 % by weight of the plant cell suspension.

7. The cosmetic product according to any one of the preceding claims, wherein the cosmetic product is provided in the form of creams, soaps, lotions, gels or hair serum.

8. The cosmetic product according to any one of the preceding claims, whereby the cosmetic product is containing an extract which is a semifinished product.

## Revendications

1. Produit cosmétique contenant un extrait d'une suspension de cellules végétales dédifférenciées provenant de plantes de la sous-famille des *Maloidae* (Malacées), de préférence *Malus domestica cultivar Uttwiler Spätlauber* (variété de pomme Uttwiler Spätlauber) pour le traitement des cellules souches pour la protection contre l'apoptose et la promotion de la prolifération, dans lequel (1) l'extrait est obtenu par homogénéisation sous haute pression d'un extrait total et (2) les composants sont extraits et stabilisés avec des liposomes, dans lequel les deux parties du déroulement du procédé sont effectuées simultanément en une seule étape de procédé.

2. Produit cosmétique selon la revendication 1, comprenant en outre des conservateurs et des antioxydants.

3. Produit cosmétique selon la revendication 2, dans lequel ledit conservateur est choisi parmi le phénoxyéthanol, l'acide benzoïque, l'acide propionique, l'alcool, le chlorure d'argent.

4. Produit cosmétique selon la revendication 2, dans lequel l'antioxydant est choisi parmi l'acide ascorbique et le tocophérol.

5. Produit cosmétique selon l'une quelconque des revendications 1 et 2, dans lequel ledit produit cosmétique présente une teneur de la suspension de cellules végétales de 0,01 à 100 % en poids.

6. Produit cosmétique selon la revendication 5, dans lequel le produit cosmétique présente une teneur de la suspension de cellules végétales de 0,1 à 10 % en poids.

7. Produit cosmétique selon l'une quelconque des revendications précédentes, sous forme de crèmes, de savons, de lotions, de gels ou de sérums capillaires.

8. Produit cosmétique selon l'une quelconque des revendications précédentes, dans lequel le produit cosmétique contient un extrait qui est un produit semi-fini.
